# EUROPEAN PATENT APPLICATION

(11) **EP 2 960 225 A1**
(43) Date of publication of application: **30.12.2015**
(21) Application number: 14754953.9
(22) Date of filing: 15.01.2014
(51) Int. Cl.: C07C 37/84, C07C 39/15

(54) **NEW 3,3',5,5'-TETRAISOPROPYL-4,4'-DIPHENOL CRYSTAL FORM AND PREPARATION METHOD THEREOF**

(30) Priority: 21.02.2013 CN 201310055828
(71) Applicant: Xi'an Libang Pharmaceutical Co., Ltd, Xi'an, Shaanxi 710075 (CN)
(72) Inventor: WANG, Rutao, Xi'an Shaanxi 710075 (CN); CHEN, Tao, Xi'an Shaanxi 710075 (CN); WANG, Weijiao, Xi'an Shaanxi 710075 (CN)
(74) Representative: Beresford, Keith Denis Lewis
(86) International application number: PCT/CN2014/070653
(87) International publication number: WO 2014/127686

(57) **Abstract**

The present invention provides 3,3',5,5'-tetraisopropyl-4,4'-diphenol crystal forms I, II and III, and preparation methods thereof. Crystal form I is superior to crystal form II in terms of stability at a high temperature, high humidity, strong light and in a water medium. The preparation methods for 3,3',5,5'-tetraisopropyl-4,4'-diphenol crystal forms I, II and III provided in the invention are simple, easy to operate and suitable for technological production.

## Description

### TECHNICAL FIELD

The present invention relates to three new crystal forms of 3,3',5,5'-tetraisopropyl-4,4'-diphenol: crystal form I, crystal form II and crystal form III, and preparation methods thereof.

### Background of Art

Epilepsy is a chronic disease where a sudden abnormal discharge in brain neurons results in a temporary brain dysfunction. According to statistics, patients with epilepsy are about 0.5% to 1% of the world's population, but currently used drugs could only partially alleviate the condition of the patients with epilepsy. 3,3',5,5'-tetraisopropyl-4,4'-diphenol is a derivative of a GABA agonist propofol. The structural formula thereof is represented by formula I.

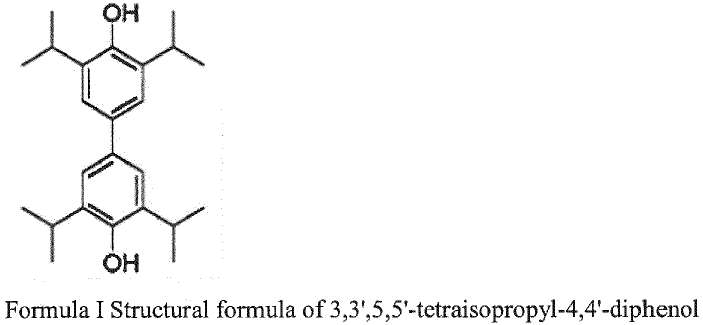

The study found that 3,3',5,5'-tetraisopropyl-4,4'-diphenol had a strong affinity for the GAGA receptor and GABA agonistic effect, and could antagonize NMDA receptors, regulate Ca2+ influx in the slow Ca2+ channel, and had a protective effect on kainate-induced excitotoxicity [1]; the antioxidant effect thereof was significantly stronger than propofol, and had a strong protective effect of brain [2 ]. And 3,3',5,5'-tetraisopropyl-4,4'-diphenol would not lead to loss of consciousness in patients, had an important clinical value for the treatment of various patients with epilepsy, and was a very promising drug.

Current research of 3,3',5,5'-tetraisopropyl-4,4'-diphenol mainly focuses on the synthesis and pharmacological activity of the compound. The literature [2, 3] reported a method for synthesizing 3,3',5,5'-tetraisopropyl-4,4'-diphenol, but did not address the problem of crystal forms; patent (application number: 201010160034.9) main described its use in the preparation of drug against epilepsy and the corresponding pharmaceutical composition, and did not study the crystal forms.

The present invention includes new crystal forms of anhydrous, monohydrate and 1,4-dioxane solvate of 3,3',5,5'-tetraisopropyl-4,4'-diphenol. Polymorph is a phenomenon that the same drug molecule is present in different crystal forms due to the different ways of crystal arrangement and filling. Since different polymorphs of the same drug have significantly different physical and chemical properties, such as solubility, dissolution rate, bioavailability, stability, flowability, compression resistance and other mechanical properties, the study of polymorphs of solid drugs becomes one of important research issues unignorable in the development of drugs.

### Summary of the Invention

The technical problem to be solved by the present invention is to provide three new crystal forms of 3,3',5,5'-tetraisopropyl-4,4'-diphenol: crystal forms I, II and III. The crystal form I of the present invention is superior to the crystal form II in the stability under light, humidity, heat and in water. The present invention further provides methods for preparing the crystal forms I, II and III of 3,3',5,5'-tetraisopropyl-4,4'-diphenol, respectively.

The present invention provides a 3,3',5,5'-tetraisopropyl-4,4'-diphenol crystal form I, which belongs to orthorhombic crystal system, where the cell parameters a = 19.9175(5) Å, b = 20.8220(6) Å, c = 10.6850(3) Å, α = β = γ = 90.00°, the unit cell volume V = 4431.3(2) Å3, and the number of asymmetric unit in the unit cell Z = 8.

The crystal form I of 3,3',5,5'-tetraisopropyl-4,4'-diphenol has characteristic peaks at diffraction angles 2θ (±0.2) = 10.22, 15.83, 17.04, 17.78, 19.18, 19.68, 20.12, 26.47, 31.35 degrees in the powder X-ray diffraction spectrum using Cu-Kα radiation source.

The present invention also provides a method for preparing the crystal form I of 3,3',5,5'-tetraisopropyl-4,4'-diphenol, comprising the steps of: (1) dissolving 3,3',5,5'-tetraisopropyl-4,4'-diphenol in an organic solvent; (2) removing the solvent by an evaporation process; (3) drying the resultant solid under vacuum to obtain 3,3',5,5'-tetraisopropyl-4,4'-diphenol crystal form I; wherein the organic solvent is selected from a single solvent of or a mixed solvent composed of: methanol, ethanol, isopropanol, n-butanol, ethyl ether, acetone, 2-butanone, ethyl acetate, nitromethane, acetonitrile, 1,4-dioxane, tetrahydrofuran, petroleum ether and n-hexane.

In step (1), the 3,3',5,5'-tetraisopropyl-4,4'-diphenol may be any form of 3,3',5,5'-tetraisopropyl-4,4'-diphenol in the art, which is available on the market.

In step (1), there is no special requirement on the amount of the organic solvent, as long as 3,3',5,5'-tetraisopropyl-4,4'-diphenol could be completely dissolved therein.

In step (1), when the organic solvent is a mixed solvent, the mixed solvent is preferably composed of two kinds of solvent, preferably in a volume ratio of 1:4 to 4:1.

In step (1), the single solvent is preferably methanol, ethanol, isopropanol, n-butanol, ethyl ether, acetone, 2-butanone, ethyl acetate, nitromethane, acetonitrile, 1,4-dioxane, tetrahydrofuran, petroleum ether; the mixed solvent is preferably ethanol and n-hexane, acetone and n-hexane, or acetonitrile and n-hexane, wherein the volume ratio of ethanol, acetone or acetonitrile to n-hexane is preferably 4:1 to 1:4; and the dissolution temperature is 15 to 40°C, more preferably 20 to 30°C.

In step (2), the evaporation temperature of the evaporation process is 25 to 60°C, preferably 25 to 40°C.

In step (2), the evaporation pressure of the evaporation process is 0.05 to 0.1MP.

In step (3), the pressure of the drying under vacuum is preferably 65 to 165 mBar.

The present invention also provides a method for preparing the crystal form I of 3,3',5,5'-tetraisopropyl-4,4'-diphenol, comprising the steps of: (1) dissolving 3,3',5,5'-tetraisopropyl-4,4'-diphenol in an organic solvent; (2) adding water thereto dropwise to precipitate a solid; (3) filtering, and drying the resultant solid under vacuum to obtain 3,3',5,5'-tetraisopropyl-4,4'-diphenol crystal form I. The organic solvent is selected from ethanol, acetone, acetonitrile, tetrahydrofuran, 1,4-dioxane.

The present invention also provides another method for preparing the crystal form I of 3,3',5,5'-tetraisopropyl-4,4'-diphenol, comprising the steps of: (1) dissolving 3,3',5,5'-tetraisopropyl-4,4'-diphenol in ethanol; (2) adding petroleum ether thereto dropwise to precipitate a solid; (3) filtering, and drying the resultant solid under vacuum to obtain 3,3',5,5'-tetraisopropyl-4,4'-diphenol crystal form I.

The present invention also provides another method for preparing the crystal form I of 3,3',5,5'-tetraisopropyl-4,4'-diphenol, comprising the step of: mechanically grinding 3,3',5,5'-tetraisopropyl-4,4'-diphenol without or with water or with anhydrous ethanol for a time to obtain 3,3',5,5'-tetraisopropyl-4,4'-diphenol crystal form I.

The present invention also provides a 3,3',5,5'-tetraisopropyl-4,4'-diphenol crystal form II, which belongs to trigonal crystal system, where the cell parameters a = 20.057(3) Å, b = 20.057(3) Å, c = 30.575(6) A, α = β = 90.00°, γ = 120°, the unit cell volume V = 10652.0(3) Å3, and the number of asymmetric unit in the unit cell Z = 18.

The crystal form II of 3,3',5,5'-tetraisopropyl-4,4'-diphenol has characteristic peaks at diffraction angles 2θ (±0.2) = 5.84, 7.68, 8.78, 11.71, 13.76, 17.77 degrees in the powder X-ray diffraction spectrum using Cu-Kα radiation source.

The present invention also provides a method for preparing the crystal form II of 3,3',5,5'-tetraisopropyl-4,4'-diphenol, comprising the steps of: (1) placing 3,3',5,5'-tetraisopropyl-4,4'-diphenol into n-heptane for suspending and agitating; (2) removing undissolved material by filtration; (3) placing the filtrate under a low temperature to precipitate a solid, to obtain 3,3',5,5'-tetraisopropyl- 4,4'-diphenol crystal form II.

The present invention also provides a method for preparing the crystal form II of 3,3',5,5'-tetraisopropyl-4,4'-diphenol, comprising the steps of: (1) dissolving 3,3',5,5'-tetraisopropyl-4,4'-diphenol in methyl t-butyl ether; (2) removing the solvent by slow evaporation; (3) drying the resultant solid under vacuum to obtain 3,3',5,5'-tetraisopropyl-4,4'-diphenol crystal form II.

The present invention also provides another method for preparing the crystal form II of 3,3',5,5'-tetraisopropyl-4,4'-diphenol, comprising the steps of: (1) dissolving 3,3',5,5'-tetraisopropyl-4,4'-diphenol in acetonitrile; (2) adding petroleum ether thereto dropwise to precipitate a solid; (3) filtering, and drying the resultant solid under vacuum to obtain 3,3',5,5'-tetraisopropyl-4,4'-diphenol crystal form II.

The present invention also provides another method for preparing the crystal form II of 3,3',5,5'-tetraisopropyl-4,4'-diphenol, comprising the steps of: (1) dissolving 3,3',5,5'-tetraisopropyl-4,4'-diphenol in a mixed solvent of ethyl acetate and n-hexane in a ratio; (2) removing the solvent by slow evaporation; (3) drying the resultant solid under vacuum to obtain 3,3',5,5'-tetraisopropyl-4,4'-diphenol crystal form II.

The present invention also provides a 3,3',5,5'-tetraisopropyl-4,4'-diphenol crystal form III, which belongs to triclinic crystal system, where the cell parameters a = 9.345(2) Å, b = 9.500(2) Å, c = 9.798(2) Å, α = 61.51(3)°, β= 82.49(3)°, γ= 62.25(3)°, the unit cell volume V = 671.9(2) Å3, and the number of asymmetric unit in the unit cell Z = 2.

The present invention also provides a method for preparing the crystal form III of 3,3',5,5'-tetraisopropyl-4,4'-diphenol, comprising the steps of: (1) dissolving 3,3',5,5'-tetraisopropyl-4,4'-diphenol in 1,4-dioxane; (2) adding thereto an organic solvent in a volume of 5 to 7 folds; (3) standing to precipitate a solid; (4) filtering, and drying the resultant solid under vacuum to obtain 3,3',5,5'-tetraisopropyl-4,4'-diphenol crystal form III. The organic solvent is selected from n-hexane and petroleum ether.

In the present invention, the aforementioned preferable conditions can be combined freely according to common knowledge in the art, to obtain preferred examples of the present invention.

The advantageous effect of the present invention:
The same drug has possibly different solubility and dissolution rate in different crystal forms, thus affecting the bioavailability of the drug; meanwhile, the physical and chemical properties of the drug, such as stability, flowability, compressibility, may be different, and these differences will eventually affect the application of the drug. This patent proposes three crystal forms of 3,3',5,5'-tetraisopropyl-4,4'-diphenol, wherein the crystal form I is the crystal form of 3,3',5,5'-tetraisopropyl-4,4'-diphenol anhydrate, the crystal form II is the crystal form of 3,3',5,5'-tetraisopropyl-4,4'-diphenol monohydrate, and the crystal form III is the crystal form of 1,4-dioxane solvate of 3,3',5,5'-tetraisopropyl-4,4'-diphenol. As compared with the solvate, the anhydrate and hydrate are more useful as pharmaceutical crystal forms. The crystal form I is the best in the stability under high temperature, high humidity, strong light, and in water medium.

### Brief Description of the Drawings

Figure 1 is X-ray powder diffraction pattern of 3,3',5,5'-tetraisopropyl-4,4'-diphenol crystal form I.
Figure 2 is DSC profile of 3,3',5,5'-tetraisopropyl-4,4'-diphenol crystal form I.
Figure 3 is X-ray powder diffraction pattern of 3,3',5,5'-tetraisopropyl-4,4'-diphenol crystal form II.
Figure 4 is DSC profile of 3,3',5,5'-tetraisopropyl-4,4'-diphenol crystal form II (Example 15).
Figure 5 is X-ray powder diffraction pattern of 3,3',5,5'-tetraisopropyl-4,4'-diphenol crystal form III.
Figure 6 is DSC profile of 3,3',5,5'-tetraisopropyl-4,4'-diphenol crystal form III.

The above description and the following examples are exemplary and illustrative only, and its protection should not be limited thereto.

### Detailed Description of Embodiment

The present invention is further described in detail below by referring to the examples, but the present invention is not limited thereto.

### Examples 1 to 14 Preparation of 3,3',5,5'-tetraisopropyl-4,4'-diphenol crystal form I

### Example 1

1 g of 3,3',5,5'-tetraisopropyl-4,4'-diphenol was dissolved in 100 mL petroleum ether at room temperature, agitated at 25°C to dissolve, and filtered. The filtrate was evaporated at 25°C under a normal pressure to remove the solvent, and then placed in an environment at 25°C to slowly volatilize, to obtain colorless, transparent, needle-like crystals, i.e., single crystals of 3,3',5,5'-tetraisopropyl-4,4'-diphenol crystal form I.

### Example 2

1 g of 3,3',5,5'-tetraisopropyl-4,4'-diphenol was dissolved in 20 mL anhydrous ethanol at room temperature, agitated at 25°C, and rapidly dissolved to give a clear solution. The solution was evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 3

1 g of 3,3',5,5'-tetraisopropyl-4,4'-diphenol was dissolved in 50 mL ethyl ether at room temperature, agitated at 25°C, and rapidly dissolved to give a clear solution. The solution was evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 4

1 g of 3,3',5,5'-tetraisopropyl-4,4'-diphenol was dissolved in 10 mL acetone at room temperature, agitated at 25°C, and rapidly dissolved to give a clear solution. The solution was evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 5

1 g of 3,3',5,5'-tetraisopropyl-4,4'-diphenol was dissolved in 10 mL ethyl acetate at room temperature, agitated at 25°C, and rapidly dissolved to give a clear solution. The solution was evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 6

1 g of 3,3',5,5'-tetraisopropyl-4,4'-diphenol was dissolved in 10 mL acetonitrile at room temperature, agitated at 25°C, and rapidly dissolved to give a clear solution. The solution was evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 7

1 g of 3,3',5,5'-tetraisopropyl-4,4'-diphenol was dissolved in 10 mL nitromethane at room temperature, agitated at 25°C, and rapidly dissolved to give a clear solution. The solution was evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 8

1 g of 3,3',5,5'-tetraisopropyl-4,4'-diphenol was dissolved in 10 mL ethanol and 40 mL n-hexane at room temperature, agitated at 25°C, and rapidly dissolved to give a clear solution. The solution was slowly evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 9

1 g of 3,3',5,5'-tetraisopropyl-4,4'-diphenol was dissolved in 25 mL acetone and 25 mL n-hexane at room temperature, agitated at 25°C, and rapidly dissolved to give a clear solution. The solution was slowly evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 10

1 g of 3,3',5,5'-tetraisopropyl-4,4'-diphenol was dissolved in 40 mL acetonitrile and 10 mL n-hexane at room temperature, agitated at 25°C, and rapidly dissolved to give a clear solution. The solution was slowly evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 11

1 g of 3,3',5,5'-tetraisopropyl-4,4'-diphenol was dissolved in 25 mL acetonitrile and 25 mL n-hexane at room temperature, agitated at 25°C, and rapidly dissolved to give a clear solution. The solution was slowly evaporated at 25°C under a normal pressure to remove the solvent, and the product was placed into a vacuum oven at 30°C and 0.1MP for drying, to obtain the crystal form I of 3,3',5,5'-tetraisopropyl-4,4'-diphenol.

### Example 12

1 g of 3,3',5,5'-tetraisopropyl-4,4'-diphenol was dissolved in 10 mL ethanol at room temperature, agitated at 25°C, and rapidly dissolved to give a clear solution. 33 mL water was slowly added to the solution at 25°C, and the solution was filtered to give a solid, which was placed into a vacuum oven at 30°C and 0.1MP for drying, to obtain the crystal form I of 3,3',5,5'-tetraisopropyl-4,4'-diphenol.

### Example 13

1 g of 3,3',5,5'-tetraisopropyl-4,4'-diphenol was dissolved in 10 mL ethanol at room temperature, agitated at 25°C, and rapidly dissolved to give a clear solution. 66 mL petroleum ether was slowly added to the solution at 25°C, and the solution was filtered to give a solid, which was placed into a vacuum oven at 30°C and 0.1MP for drying, to obtain the crystal form I of 3,3',5,5'-tetraisopropyl-4,4'-diphenol.

### Example 14

1 g of 3,3',5,5'-tetraisopropyl-4,4'-diphenol was placed into a mortar, 500µL anhydrous ethanol was added thereto, and grinding was carried out for 5 minutes to give the crystal form I of 3,3',5,5'-tetraisopropyl-4,4'-diphenol.

### Examples 15 to 18 Preparation of 3,3',5,5'-tetraisopropyl-4,4'-diphenol crystal form II

### Example 15

1g of 3,3',5,5'-tetraisopropyl-4,4'-diphenol was placed into 120mL n-heptane for suspending and agitating at room temperature, and filtered. The filtrate was placed under a condition of -20°C for 12h to give a colorless, transparent crystalline block, i.e., single crystals of 3,3',5,5'-tetraisopropyl-4,4'-diphenol crystal form II.

### Example 16

1 g of 3,3',5,5'-tetraisopropyl-4,4'-diphenol was dissolved in 50 mL methyl t-butyl ether at room temperature, and agitated and dissolved at 25°C to give a clear solution. The solution was slowly evaporated at 25°C under a normal pressure to remove the solvent, and the product was placed into a vacuum oven at 30°C and 0.1MP for drying, to obtain the crystal form II of 3,3',5,5'-tetraisopropyl-4,4'-diphenol.

### Example 17

1 g of 3,3',5,5'-tetraisopropyl-4,4'-diphenol was dissolved in 5 mL acetonitrile and 67 mL petroleum ether at room temperature, agitated at 25°C, and rapidly dissolved to give a clear solution. The solution was slowly evaporated at 25°C under a normal pressure to remove the solvent, and the product was placed into a vacuum oven at 30°C and 0.1MP for drying, to obtain the crystal form II of 3,3',5,5'-tetraisopropyl-4,4'-diphenol.

### Example 18

1 g of 3,3',5,5'-tetraisopropyl-4,4'-diphenol was dissolved in 40 mL n-hexane and 10 mL ethyl acetate at room temperature, agitated at 25°C, and rapidly dissolved to give a clear solution. The solution was slowly evaporated at 25°C under a normal pressure to remove the solvent, and the product was placed into a vacuum oven at 30°C and 0.1MP for drying, to obtain the crystal form II of 3,3',5,5'-tetraisopropyl-4,4'-diphenol.

### Examples 19 and 20 Preparation of 3,3',5,5'-tetraisopropyl-4,4'-diphenol crystal form III

1 g of 3,3',5,5'-tetraisopropyl-4,4'-diphenol was placed into 5 mL 1,4-dioxane at room temperature, agitated at 25°C, and rapidly dissolved to give a clear solution. 30 mL petroleum ether was slowly added to the solution, which was then allowed to stand for 1h to precipitate a solid, to obtain colorless, transparent, needle-like crystals, i.e., single crystals of 3,3',5,5'-tetraisopropyl-4,4'-diphenol crystal form III.

### Example 20

1 g of 3,3',5,5'-tetraisopropyl-4,4'-diphenol was dissolved in 5 mL 1,4-dioxane at room temperature, agitated at 25°C, and rapidly dissolved to give a clear solution. 30 mL n-hexane was slowly added thereto under agitation at 25°C, and the solution was allowed to stand for 1h to precipitate a solid. The solid is filtered out, and placed into a vacuum oven at 30°C and 0.1MP for drying, to obtain the crystal form III of 3,3',5,5'-tetraisopropyl-4,4'-diphenol.

### Detection and analysis of crystal form I

### Effect Example 1 3,3',5,5'-tetraisopropyl-4,4'-diphenol crystal form I

The single crystal of crystal form I obtained from Example 1 was subjected to a single crystal X-ray diffraction analysis. The crystal thereof belongs to orthorhombic crystal system, where the cell parameters a = 19.9175(5) Å, b = 20.8220(6) Å, c = 10.6850(3) Å, α = β = γ = 90.00°, the unit cell volume V = 4431.3(2) Å3, and the number of asymmetric unit in the unit cell Z = 8.

### Effect Example 2

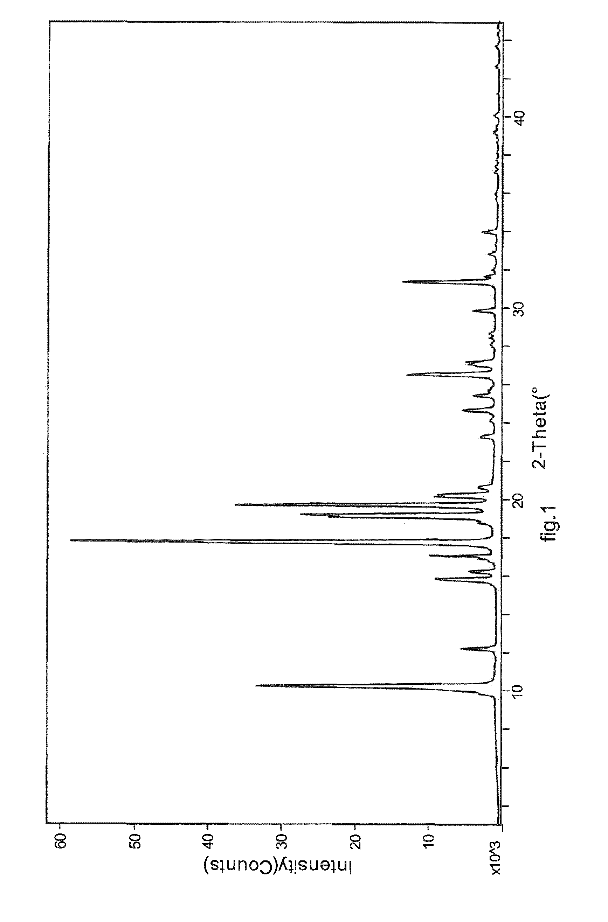

The crystal form I of 3,3',5,5'-tetraisopropyl-4,4'-diphenol obtained from Example 6 was subjected to a powder X-ray diffraction analysis with a radiation source of Cu-Kα. The pattern thereof was shown in Figure 1, and specific characteristic peaks were listed in Table 1.

**Table 1 PXRD characteristic peaks of crystal form I of 3,3',5,5'-tetraisopropyl-4,4'-diphenol**

| No. | 2θ angle (°) | Height | Peak intensity% |
|---|---|---|---|
| 1 | 10.224 | 32470 | 56.7 |
| 2 | 12.180 | 4831 | 8.4 |
| 3 | 15.829 | 7977 | 13.9 |
| 4 | 16.223 | 3511 | 6.1 |
| 5 | 17.036 | 8114 | 14.2 |
| 6 | 17.782 | 57291 | 100.0 |
| 7 | 18.754 | 1923 | 3.4 |
| 8 | 19.183 | 25567 | 44.6 |
| 9 | 19.680 | 34394 | 60.0 |
| 10 | 20.116 | 6851 | 12.0 |
| 11 | 20.591 | 1435 | 2.5 |
| 12 | 23.217 | 1816 | 3.2 |
| 13 | 24.138 | 603 | 1.1 |
| 14 | 24.633 | 4275 | 7.5 |
| 15 | 25.423 | 2750 | 4.8 |
| 16 | 25.678 | 774 | 1.4 |
| 17 | 26.473 | 11679 | 20.4 |
| 18 | 27.176 | 3787 | 6.6 |
| 19 | 28.124 | 628 | 1.1 |
| 20 | 28.445 | 763 | 1.3 |
| 21 | 28.697 | 768 | 1.3 |
| 22 | 29.844 | 3036 | 5.3 |
| 23 | 31.345 | 12515 | 21.8 |
| 24 | 31.639 | 1521 | 2.7 |
| 25 | 32.823 | 1075 | 1.9 |
| 26 | 33.968 | 2051 | 3.6 |
| 27 | 40.068 | 699 | 1.2 |
| 28 | 42.631 | 533 | 0.9 |

The crystal form I obtained from other Examples (1-5, 7-14) has the same X-ray diffraction patterns and characteristic peaks as those in Example 6.

### Effect Example 3

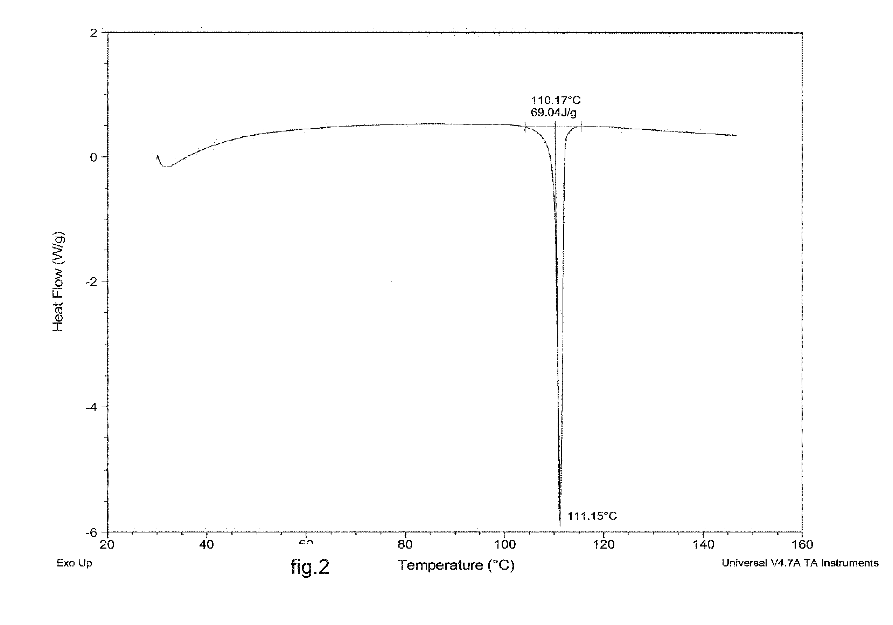

The crystal form I of 3,3',5,5'-tetraisopropyl-4,4'-diphenol from Example 6 was detected by differential scanning calorimetry (DSC), in which the temperature was raised to 150°C from 30°C at a rate of 10°C/min. There was an endothermic peak at 111±2°C, see Figure 2 for details. The DSC profiles of other examples (1-5, 7-14) were the same as those in this Example.

### Detection and analysis of crystal form II

### Effect Example 4 3,3',5,5'-tetraisopropyl-4,4'-diphenol crystal form II

The single crystal of crystal form II obtained from Example 15 was subjected to a single crystal X-ray diffraction analysis. The crystal thereof belongs to trigonal crystal system, where the cell parameters a = 20.057(3) Å, b = 20.057(3) Å, c = 30.575(6) A, α = β = 90.00°, γ = 120°, the unit cell volume V = 10652.0(3) Å3, and the number of asymmetric unit in the unit cell Z = 18.

### Effect Example 5

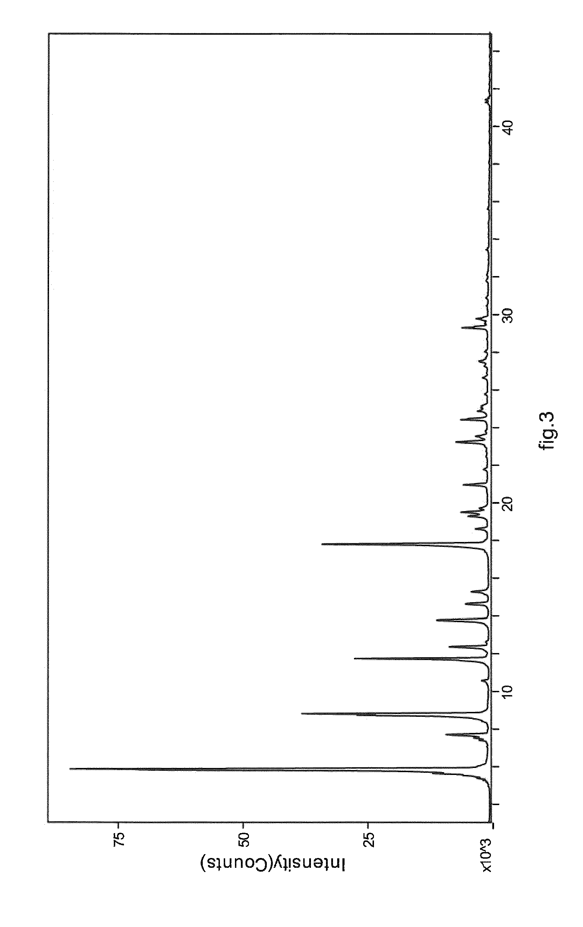

The crystal form II of 3,3',5,5'-tetraisopropyl-4,4'-diphenol obtained from Example 15 was subjected to a powder X-ray diffraction analysis with a radiation source of Cu-Kα. The pattern thereof was shown in Figure 3, and specific characteristic peaks were listed in Table 2.

**Table 2 PXRD characteristic peaks of crystal form II of 3,3',5,5'-tetraisopropyl-4,4'-diphenol**

| No. | 2θ angle (°) | Height | Peak intensity% |
|---|---|---|---|
| 1 | 5.840 | 84424 | 100 |
| 2 | 7.501 | 2895 | 3.4 |
| 3 | 7.675 | 8410 | 10.0 |
| 4 | 8.780 | 37382 | 44.3 |
| 5 | 10.554 | 1423 | 1.7 |
| 6 | 11.705 | 26890 | 31.9 |
| 7 | 12.337 | 7793 | 9.2 |
| 8 | 12.596 | 437 | 0.5 |
| 9 | 13.756 | 10366 | 12.3 |
| 10 | 14.607 | 4641 | 5.5 |
| 11 | 15.258 | 3446 | 4.1 |
| 12 | 17.769 | 33274 | 39.4 |
| 13 | 18.595 | 2395 | 2.8 |
| 14 | 19.268 | 3888 | 4.6 |
| 15 | 19.483 | 5367 | 6.4 |
| 16 | 19.696 | 1631 | 1.9 |
| 17 | 20.928 | 4909 | 5.8 |
| 18 | 21.754 | 813 | 1.0 |
| 19 | 23.210 | 6303 | 7.5 |
| 20 | 23.525 | 2255 | 2.7 |
| 21 | 23.798 | 296 | 0.4 |
| 22 | 24.402 | 5337 | 6.3 |
| 23 | 24.855 | 1969 | 2.3 |
| 24 | 25.125 | 1243 | 1.5 |
| 25 | 25.748 | 531 | 0.6 |
| 26 | 26.647 | 983 | 1.2 |
| 27 | 27.263 | 705 | 0.8 |
| 28 | 27.531 | 1709 | 2.0 |
| 29 | 28.032 | 570 | 0.7 |
| 30 | 29.296 | 5246 | 6.2 |
| 31 | 29.785 | 2365 | 2.8 |
| 32 | 30.873 | 446 | 0.5 |
| 33 | 31.765 | 432 | 0.5 |
| 34 | 32.098 | 365 | 0.4 |
| 35 | 33.436 | 539 | 0.6 |
| 36 | 41.412 | 864 | 1.0 |

The crystal form II obtained from Example 16-18 has the same X-ray diffraction pattern and characteristic peaks as those in this Example.

### Effect Example 6

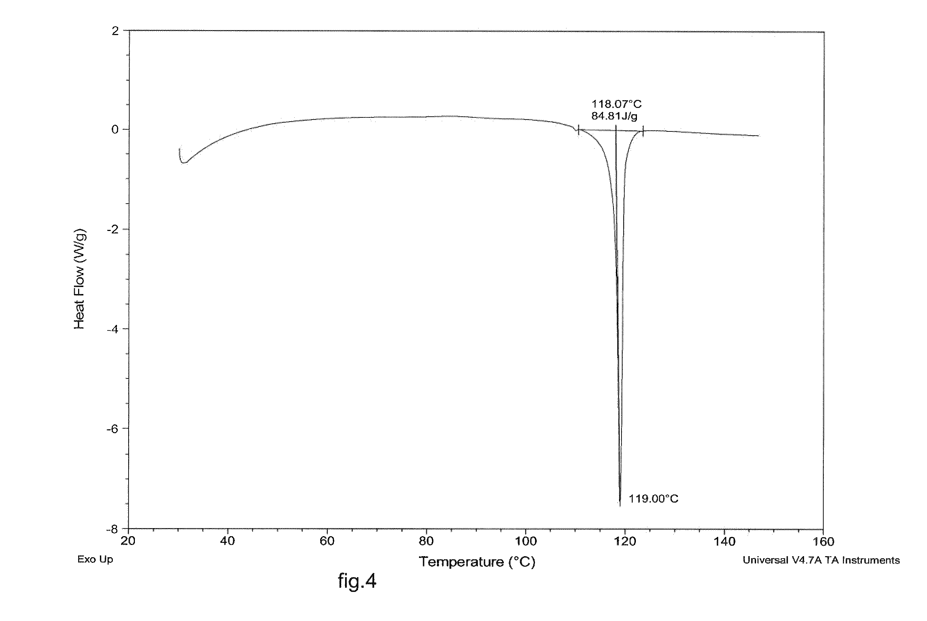

The crystal form II of 3,3',5,5'-tetraisopropyl-4,4'-diphenol from Example 15 was detected by differential scanning calorimetry (DSC), in which the temperature was raised to 150°C from 30°C at a rate of 10°C/min. There was an endothermic peak at 119±2°C, see Figure 4 for details.

### Detection and analysis of crystal form III

### Effect Example 7 3,3',5,5'-tetraisopropyl-4,4'-diphenol crystal form III

The single crystal of crystal form III obtained from Example 19 was subjected to a single crystal X-ray diffraction analysis. The crystal thereof belongs to triclinic crystal system, where the cell parameters a = 9.345(2) Å, b = 9.500(2) A, c = 9.798(2) Å, α = 61.51(3)°, β= 82.49(3)°, γ= 62.25(3)°, the unit cell volume V = 671.9(2) Å3, and the number of asymmetric unit in the unit cell Z = 2.

### Effect Example 8

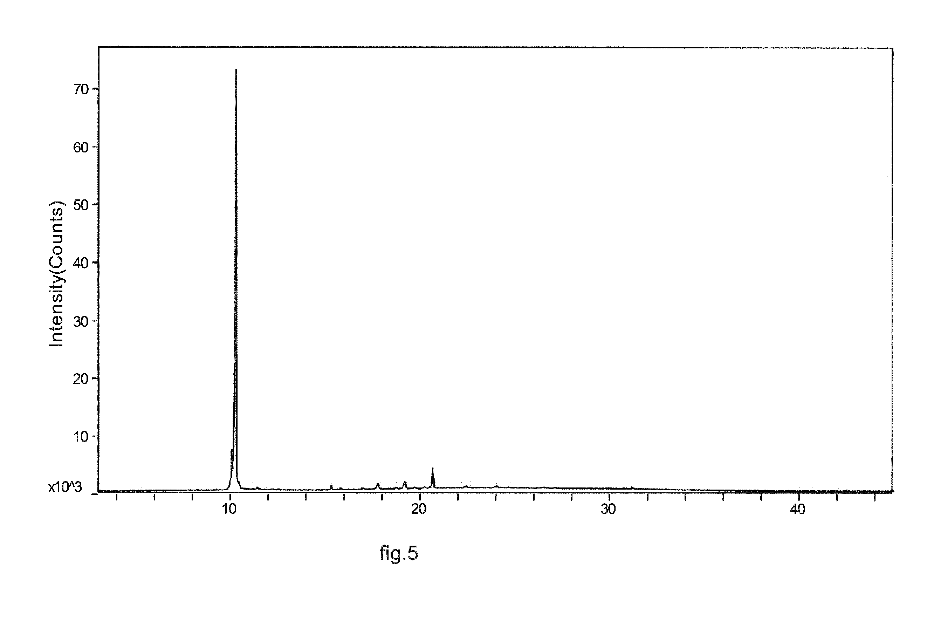

The crystal form III of 3,3',5,5'-tetraisopropyl-4,4'-diphenol obtained from Example 20 was subjected to a powder X-ray diffraction analysis with a radiation source of Cu-Kα. The pattern thereof was shown in Figure 5, and specific characteristic peaks were listed in Table 3.

**Table3 PXRD characteristic peaks of crystal form III of 3,3',5,5'-tetraisopropyl-4,4'-diphenol**

| No. | 2θ angle (°) | Height | Peak intensity% |
|---|---|---|---|
| 1 | 10.259 | 72913 | 100.0 |
| 2 | 11.409 | 371 | 0.5 |
| 3 | 15.295 | 353 | 0.5 |
| 4 | 15.808 | 205 | 0.3 |
| 5 | 16.972 | 238 | 0.3 |
| 6 | 17.747 | 860 | 1.2 |
| 7 | 18.702 | 243 | 0.3 |
| 8 | 19.183 | 1166 | 1.6 |
| 9 | 19.686 | 236 | 0.3 |
| 10 | 20.232 | 168 | 0.2 |
| 11 | 20.650 | 3464 | 4.8 |
| 12 | 22.419 | 326 | 0.4 |
| 13 | 24.007 | 322 | 0.4 |
| 14 | 26.549 | 180 | 0.2 |
| 15 | 29.925 | 196 | 0.3 |
| 16 | 31.194 | 319 | 0.4 |

Example 19 has the same pattern and characteristic peaks as those in this Example.

### Effect Example 9

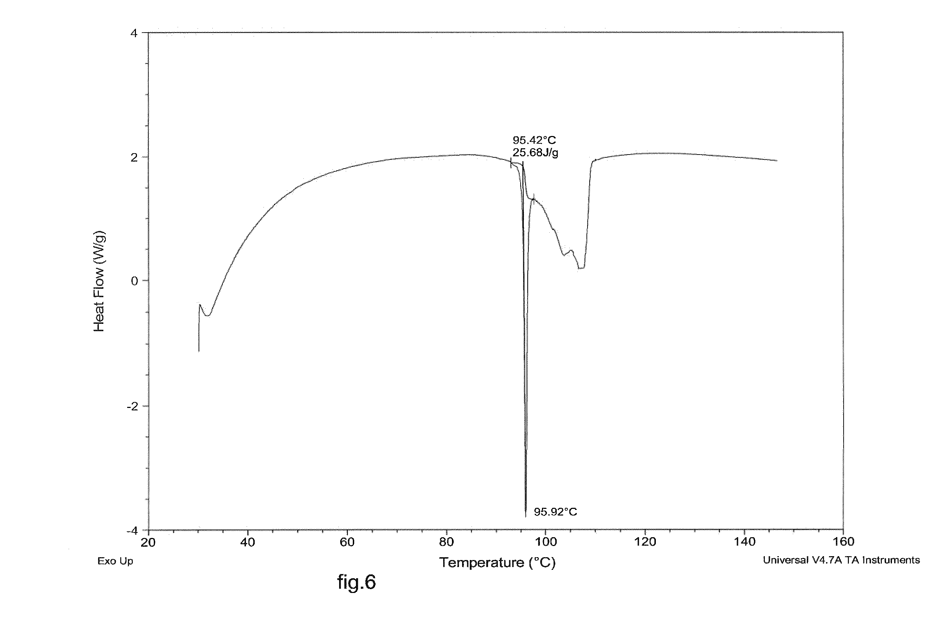

The crystal form III of 3,3',5,5'-tetraisopropyl-4,4'-diphenol from Example 20 was detected by differential scanning calorimetry (DSC), in which the temperature was raised to 150°C from 30°C at a rate of 10°C/min. There was an endothermic peak at 96±2°C, see Figure 6 for details.

The DSC profile of Example 19 was the same as this Example.

### Effect Example 10

As compared with the solvate, the anhydrate and hydrate are more useful as pharmaceutical crystal forms. The relative stability of the crystal form is an important indicator to be investigated in the research of drug polymorph, and the stability under high temperature, high humidity, strong light, and in water is investigated for the crystal forms I and II. The result shows that the crystal form I is the best in the stability under high temperature, high humidity, strong light, and in water medium.

### 1. High temperature

The crystal forms I and II prepared in Example 6 and 15 were respectively placed in a ventilated oven at 80°C for 7 days and then characterized, and the result was shown in Table 4. As seen from the table, under high temperature conditions, the crystal form II has a tendency of converting to the crystal form I.

### 2. High humidity

The crystal forms I and II prepared in Example 6 and 15 were respectively placed in a constant temperature and humidity chamber at 80°C and 75% RH for 7 days and then characterized, and the result was shown in Table 4. As seen from the table, under high humidity conditions, the crystal form II has a tendency of converting to the crystal form I.

### 3. Strong light

The crystal forms I and II prepared in Example 6 and 15 were respectively placed in a light stable box with illumination of 4500lux for 7 days and then characterized, and the result was shown in Table 4. As seen from the table, under strong light conditions, the crystal form I and II have a good stability, and interconversion phenomenon does not appear.

### 4. Water

The crystal forms I and II prepared in Example 6 and 15 were placed in water at 25°C and suspended for 7 days and then characterized, and the result was shown in Table 4. As seen from the table, in water medium, the crystal form II has a tendency of converting to the crystal form I.

**Table 4 Experimental result of stability of crystal forms**

| Original crystal form | High temperature (80°C) | High humidity (40°C, 75% RH) | Light | Water |
|---|---|---|---|---|
| I | I | I | I | I |
| II | I, II | I (few), II | II | I (few), II |

## Claims

1. A 3,3',5,5'-tetraisopropyl-4,4'-diphenol crystal form I, **characterized in that** the crystal belongs to orthorhombic crystal system, where the cell parameters a = 19.9175(5) A, b = 20.8220(6) Å, c = 10.6850(3) A, α = β = γ = 90.00°, the unit cell volume V = 4431.3(2) Å3, and the number of asymmetric unit in the unit cell Z = 8.

2. The crystal form according to claim 1, further **characterized in that** it has characteristic peaks at diffraction angles 2θ (±0.2) = 10.22, 15.83, 17.04, 17.78, 19.18, 19.68 , 20.12, 26.47, 31.35 degrees in the powder X-ray diffraction spectrum using Cu-Kα radiation source.

3. A method for preparing the crystal form I according to claim 1 or 2, comprising the steps of: (1) dissolving 3,3',5,5'-tetraisopropyl-4,4'-diphenol in an organic solvent; (2) removing the solvent by an evaporation process; (3) drying the resultant solid under vacuum to obtain 3,3',5,5'-tetraisopropyl-4,4'-diphenol crystal form I; wherein the organic solvent is selected from a single solvent of or a mixed solvent composed of: methanol, ethanol, isopropanol, n-butanol, ethyl ether, acetone, 2-butanone, ethyl acetate, nitromethane, acetonitrile, 1,4-dioxane, tetrahydrofuran, petroleum ether and n-hexane.

4. The method according to claim 3, **characterized in that** in step (1), the single solvent is methanol, ethanol, isopropanol, n-butanol, ethyl ether, acetone, 2-butanone, ethyl acetate, nitromethane, acetonitrile, 1,4-dioxane, tetrahydrofuran, or petroleum ether; the mixed solvent is ethanol and n-hexane, acetone and n-hexane, or acetonitrile and n-hexane, wherein the volume ratio of ethanol, acetone or acetonitrile to n-hexane is preferably 4:1 to 1:4; and the dissolution temperature is 15 to 40°C, more preferably 20 to 30°C.

5. The method according to claim 3, **characterized in that** in step (2), the evaporation temperature of the evaporation process is 25 to 60°C, preferably 25 to 40°C; and the evaporation pressure of the evaporation process is 0.05 to 0.1MP.

6. The method according to claim 3, **characterized in that** in step (3), the pressure of the drying under vacuum is preferably 65 to 165 mBar.

7. A method for preparing the crystal form I according to claim 1 or 2, comprising the steps of: (1) dissolving 3,3',5,5'-tetraisopropyl-4,4'-diphenol in an organic solvent; (2) adding water thereto dropwise to precipitate a solid; (3) filtering, and drying the resultant solid under vacuum to obtain 3,3',5,5'-tetraisopropyl-4,4'-diphenol crystal form I; wherein the organic solvent is selected from ethanol, acetone, acetonitrile, tetrahydrofuran, 1,4-dioxane.

8. A method for preparing the crystal form I according to claim 1 or 2, comprising the steps of: (1) dissolving 3,3',5,5'-tetraisopropyl-4,4'-diphenol in ethanol; (2) adding petroleum ether thereto dropwise to precipitate a solid; (3) filtering, and drying the resultant solid under vacuum to obtain 3,3',5,5'-tetraisopropyl-4,4'-diphenol crystal form I.

9. A method for preparing the crystal form I according to claim 1 or 2, comprising the step of: mechanically grinding 3,3',5,5'-tetraisopropyl-4,4'-diphenol without or with water or with anhydrous ethanol for a time to obtain 3,3',5,5'-tetraisopropyl-4,4'-diphenol crystal form I.

10. A 3,3',5,5'-tetraisopropyl-4,4'-diphenol crystal form II, **characterized in that** the crystal belongs to trigonal crystal system, where the cell parameters a = 20.057(3) Å, b = 20.057(3) Å, c = 30.575(6) A, α = β = 90.00°, γ = 120°, the unit cell volume V = 10652.0(3) Å3, and the number of asymmetric unit in the unit cell Z = 18.

11. The crystal form II according to claim 10, **characterized in that** it has characteristic peaks at diffraction angles 2θ (±0.2) = 5.84, 7.68, 8.78, 11.71, 13.76, 17.77 degrees in the powder X-ray diffraction spectrum using Cu-Kα radiation source.

12. A method for preparing the crystal form II according to claim 10 or 11, comprising the steps of: (1) placing 3,3',5,5'-tetraisopropyl-4,4'-diphenol into n-heptane for suspending and agitating; (2) removing undissolved material by filtration; (3) placing the filtrate under a low temperature to precipitate a solid, to obtain 3,3',5,5'-tetraisopropyl- 4,4'-diphenol crystal form II.

13. A method for preparing the crystal form II according to claim 10 or 11, comprising the steps of: (1) dissolving 3,3',5,5'-tetraisopropyl-4,4'-diphenol in methyl t-butyl ether; (2) removing the solvent by slow evaporation; (3) drying the resultant solid under vacuum to obtain 3,3',5,5'-tetraisopropyl-4,4'-diphenol crystal form II.

14. A method for preparing the crystal form II according to claim 10 or 11, comprising the steps of: (1) dissolving 3,3',5,5'-tetraisopropyl-4,4'-diphenol in acetonitrile; (2) adding petroleum ether thereto dropwise to precipitate a solid; (3) filtering, and drying the resultant solid under vacuum to obtain 3,3',5,5'-tetraisopropyl-4,4'-diphenol crystal form II.

15. A method for preparing the crystal form II according to claim 10 or 11, comprising the steps of: (1) dissolving 3,3',5,5'-tetraisopropyl-4,4'-diphenol in a mixed solvent of ethyl acetate and n-hexane in a ratio; (2) removing the solvent by slow evaporation; (3) drying the resultant solid under vacuum to obtain 3,3',5,5'-tetraisopropyl-4,4'-diphenol crystal form II.

16. A 3,3',5,5'-tetraisopropyl-4,4'-diphenol crystal form III, **characterized in that** the crystal belongs to triclinic crystal system, where the cell parameters a = 9.345(2) A, b = 9.500(2) Å, c = 9.798(2) Å, α = 61.51(3)°, β= 82.49(3)°, γ= 62.25(3)°, the unit cell volume V = 671.9(2) Å3, and the number of asymmetric unit in the unit cell Z = 2.

17. A method for preparing the crystal form III according to claim 16, comprising the steps of: (1) dissolving 3,3',5,5'-tetraisopropyl-4,4'-diphenol in 1,4-dioxane; (2) adding thereto an organic solvent in a volume of 5 to 7 folds; (3) standing to precipitate a solid; (4) filtering, and drying the resultant solid under vacuum to obtain 3,3',5,5'-tetraisopropyl-4,4'-diphenol crystal form III, wherein the organic solvent is selected from n-hexane and petroleum ether.
